**Europäisches Patentamt**

**European Patent Office**

**Office européen des brevets**

(19)

(11) Numéro de publication: **0 143 017**
**B1**

(12) # FASCICULE DE BREVET EUROPEEN

(45) Date de publication du fascicule du brevet:
**10.12.86**

(51) Int. Cl.⁴: **C 07 C 45/29**, C 07 C 49/713

(21) Numéro de dépôt: **84401862.2**

(22) Date de dépôt: **20.09.84**

(54) **Procédé de préparation de l'hydroxy-4 triméthyl-2,4,6 cyclohexadiène-2,5 one.**

(30) Priorité: **22.09.83 FR 8315071**

(43) Date de publication de la demande:
**29.05.85 Bulletin 85/22**

(45) Mention de la délivrance du brevet:
**10.12.86 Bulletin 86/50**

(84) Etats contractants désignés:
**AT BE CH DE FR GB IT LI LU NL SE**

(56) Documents cités:
**FR - A - 1 434 526**
**FR - A - 2 258 361**

(73) Titulaire: **RHONE-POULENC SANTE, Les Miroirs 18 Avenue d'Alsace, F-92400 Courbevoie Cedex (FR)**

(72) Inventeur: **Costantini, Michel, 9 place Aristide Briand, F-69003 Lyon (FR)**
Inventeur: **Igersheim, Françoise, 21 rue Florian, F-69100 Villeurbanne (FR)**
Inventeur: **Krumenacker, Léon, 2 allée du Bois-Rond, F-69360 Serezin Du Rhone (FR)**

(74) Mandataire: **Pilard, Jacques et al, RHONE-POULENC RECHERCHES Service Brevets Pharma 25, Quai Paul Doumer, F-92408 Courbevoie Cedex (FR)**

## Description

La présente invention concerne un nouveau procédé de préparation de l'hydroxy-4 triméthyl-2,4,6 cyclohexadiène-2,5 one qui constitue un intermédiaire pour la synthèse de la triméthylhydroquinone (TMHQ) qui est elle-même un précurseur de la vitamine E.

Il est connu de préparer l'hydroxy-4 triméthyl-2,4,6 cyclohexadiène-2,5 one par oxydation du triméthyl-2,4,6 phénol au moyen par exemple d'un peracide ou d'oxygène moléculaire en milieu basique. Cependant l'oxydation à l'air sous une pression voisine de 100 bars présente des difficultés de réalisation technique liées à des problèmes importants de sécurité.

L'hydroxy-4 triméthyl-2,4,6 cyclohexadiène-2,5 one peut être transformée en triméthylhydroquinone dans les conditions décrites dans le brevet français N° 73 33374 publié sous le numéro 2 200 225, c'est-à-dire par chauffage à une température d'au moins 100°C dans un milieu liquide non acide tel que le méthanol en milieu aqueux.

Il est connu également d'après le brevet français N° 70 23875 publié sous le numéro 2 051 407 de préparer la triméthylhydroquinone par sulfonation du triméthyl-2,3,6 phénol, oxyydation de l'acide triméthyl-2,3,6 phénolsulfonique-4 formé puis réduction immédiate de la triméthylquinone résultante.

La sulfonation est généralement effectuée au moyen d'acide sulfurique concentré à une température inférieure ou égale à 60°C. L'oxydation est réalisée au moyen d'un agent oxydant choisi parmi l'acide chromique, le bioxyde de manganèse ou le sulfate manganique à une température inférieure ou égale à la température d'ébullition du mélange réactionnel. La réduction est, de préférence, réalisée au moyen de dithionite de sodium.

Il a maintenant été trouvé, et c'est ce qui fait l'objet de la présente invention, que l'hydroxy-4 triméthyl-2,4,6 cyclohexadiène-2,5 one pouvait être obtenue par oxydation du triméthyl-2,4,6 phénol au moyen d'un dérivé du manganèse de valence supérieure à 2 en milieu acide.

Comme dérivés du manganèse de valence supérieure à 2 peuvent être cités le bioxyde de manganèse, le permanganate de potassium ou le sulfate manganique ou leurs mélanges. Il est particulièrement avantageux d'utiliser le bioxyde de manganèse comme agent d'oxydation.

Généralement, la réaction d'oxydation est réalisée en présence d'un acide fort tel que l'acide sulfurique, l'acide perchlorique ou les acides sulfoniques (acide méthanesulfonique, acide p.toluènesulfonique). Il est particulièrement avantageux d'opérer dans une phase aqueuse dont le pH est compris entre −1 et +1 et de préférence entre −0,8 et +0,5.

Le procédé selon l'invention peut être mis en œuvre en ajoutant le dérivé du manganèse au triméthyl-2,4,6 phénol en suspension en milieu acide aqueux ou en solution dans un solvant organique en contact avec le milieu acide aqueux. Le dérivé du manganèse peut être ajouté sous forme solide ou sous forme d'une suspension ou d'une solution aqueuse.

Le procédé peut aussi être mis en œuvre en ajoutant le triméthyl-2,4,6 phénol éventuellement en solution dans un solvant organique à une suspension ou une solution du dérivé du manganèse en milieu acide aqueux contenant éventuellement un solvant organique.

Lorsque le procédé est mis en œuvre en présence d'un solvant organique, celui-ci est choisi parmi les hydrocarbures aromatiques (benzène), les hydrocarbures aliphatiques (hexane) ou cycloaliphatiques (cyclohexane), les éthers (diisopropyléther), les esters (acétate d'éthyle), les solvants chlorés (tétrachlorure de carbone) ou les acides organiques (acide acétique).

Lorsque l'on utilise comme agent d'oxydation le bioxyde de manganèse, il est particulièrement avantageux d'utiliser un rapport moléculaire du bioxyde de manganèse au triméthyl-2,4,6 phénol compris entre 1 et 2 et de préférence voisin de 1,5.

Généralement, le procédé est mis en œuvre à une température comprise entre −10°C et la température d'ébullition du mélange réactionnel et de préférence entre −10 et 30°C.

Le procédé peut également être mis en œuvre en ajoutant progressivement l'acide fort à un mélange du dérivé du manganèse et du triméthyl-2,4,6 phénol dans l'eau acidifiée à un pH voisin de −0,6 en présence éventuellement d'un solvant organique de façon à maintenir le pH voisin de −0,6.

Lorsque l'on opère en absence d'un solvant organique, l'hydroxy-4 triméthyl-2,4,6 cyclohexadiène-2,5 one peut être isolée, après extraction du mélange réactionnel par un solvant organique non miscible à l'eau.

Lorsque l'on opère en présence d'un solvant organique, l'hydroxy-4 triméthyl-2,4,6 cyclohexadiène-2,5 one peut être isolée après décantation puis extraction de la phase aqueuse par un solvant organique non miscible à l'eau.

Dans l'un ou l'autre cas, l'hydroxy-4 triméthyl-2,4,6 cyclohexadiène-2,5 one est obtenue en solution dans le solvant organique qu'il suffit d'évaporer pour isoler le produit.

Il est possible de purifier l'hydroxy-4 triméthyl-2,4,6 cyclohexadiène-2,5 one à partir de sa solution dans un solvant organique après lavage de celle-ci à l'eau puis extraction de la phase aqueuse par un solvant organique non miscible à l'eau. L'hydroxy-4 triméthyl-2,4,6 cyclohexadiène-2,5 one est isolée après évaporation du solvant et éventuellement après distillation.

En opérant selon le procédé de la présente invention, le taux de transformation du triméthyl-2,4,6 phénol est voisin de 100% et le rendement en hydroxy-4 triméthyl-2,4,6 cyclohexadiène-2,5 one est généralement compris entre 65 et 70%.

Les exemples suivants, donnés à titre non limitatif, montrent comment l'invention peut être mise en pratique.

*Exemple 1 :*

Dans un ballon de 500 cm³, muni d'une agitation centrale, d'une ampoule de coulée, d'un réfrigérant ascendant et d'un thermomètre, on introduit 3,9 g de bioxyde de manganèse (37,1 m.moles), 170 cm³ d'eau et 30 g d'acide sulfurique concentré (d = 1,83). Le mélange est refroidi à une température voisine de 0°C. On ajoute 100 cm³ d'éther isopropylique. On ajoute ensuite, en 30 minutes, 3,4 g de triméthyl-2,4,6 phénol (25 m.moles) en solution dans 60 cm³ d'éther isopropylique. L'ampoule de coulée est rincée par 25 cm³ d'éther isopropylique. Après 5 heures 30 d'agitation à une température comprise entre 0 et 5°C, le mélange réactionnel est décanté. La phase aqueuse est extraite par 3 fois 25 cm³ d'éther isopropylique. Dans les phases organiques réunies, on dose, par chromatographie en phase gazeuse, 0,65 m.moles de triméthyl-2,4,6 phénol et 16 m.moles d'hydroxy-4 triméthyl-2,4,6 cyclohexadiène-2,5 one.

Le taux de transformation du triméthyl-2,4,6 phénol est de 97,4% et le rendement en hydroxy-4 triméthyl-2,4,6 cyclohexadiène-2,5 one est de 65,7% par rapport au triméthyl-2,4,6 phénol transformé.

*Exemple 2:*

On opère comme dans l'exemple 1 mais en remplaçant l'éther isopropylique par les mêmes volumes de tétrachlorure de carbone. 3 heures après la fin de l'addition du triméthyl-2,4,6 phénol, le mélange réactionnel est décanté et la phase aqueuse est extraite par 3 fois 25 cm³ d'éther isopropylique. Dans les phases organiques réunies, on dose, par chromatographie en phase gazeuse, 0,5 m.mole de triméthyl-2,4,6 phénol et 16,8 m.moles d'hydroxy-4 triméthyl-2,4,6 cyclohexadiène-2,5 one.

Le taux de transformation du triméthyl-2,4,6 phénol est de 98% et le rendement en hydroxy-4 triméthyl-2,4,6 cyclohexadiène-2,5 one est de 68,7% par rapport au triméthyl-2,4,6 phénol transformé.

*Exemple 3:*

Dans l'appareillage analogue à celui décrit dans l'exemple 1, mais en utilisant un ballon de 2 litres, on introduit 15,74 g de bioxyde de manganèse (150 m.moles), 680 cm³ d'eau et 120 g d'acide sulfurique concentré (d = 1,83). On refroidit à 0°C puis on ajoute 100 cm³ de tétrachlorure de carbone. On ajoute ensuite, en 45 minutes, 13,6 g de triméthyl-2,4,6 phénol (100 m.moles) en solution dans 80 cm³ de tétrachlorure de carbone. L'ampoule de coulée est rincée par 20 cm³ de tétrachlorure de carbone.

Après 4 heures d'agitation à une température comprise entre 0 et 5°C, le mélange réactionnel est décanté et la phase aqueuse est extraite par 6 fois 100 cm³ d'éther isopropylique. Dans les phases organiques réunies, on dose, par chromatographie en phase gazeuse, 3,9 m.moles de triméthyl-2,4,6 phénol et 66,4 m.moles d'hydroxy-4 triméthyl-2,4,6 cyclohexadiène-2,5 one.

Le taux de transformation du triméthyl-2,4,6 phénol est de 95,9% et le rendement en hydroxy-4 triméthyl-2,4,6 cyclohexadiène-2,5 one est de 69,1% par rapport au triméthyl-2,4,6 phénol transformé.

*Exemple 4:*

Dans un ballon de 250 cm³ muni d'une agitation centrale, d'une ampoule de coulée, d'un réfrigérant ascendant et d'un thermomètre, on introduit 3,96 g de bioxyde de manganèse (37,7 m.moles), 44,5 cm³ d'eau et 10 g d'acide sulfurique concentré (d = 1,83). On refroidit au voisinage de 0°C puis on ajoute 20 cm³ de tétrachlorure de carbone. On ajoute ensuite, en 16 minutes, 3,4 g de triméthyl-2,4,6 phénol (25 m.moles) en solution dans 25 cm³ de tétrachlorure de carbone. L'ampoule de coulée est rincée par 5 cm³ de tétrachlorure de carbone. Après 4 heures 10 d'agitation à une température comprise entre 0 et 5°C, le mélange réactionnel est décanté et la couche aqueuse est extraite par 3 fois 25 cm³ de tétrachlorure de carbone. Dans les phases organiques réunies, on dose, par chromatographie en phase gazeuse, 1,3 m.mole de triméthyl-2,4,6 phénol et 10,8 m.moles d'hydroxy-4 triméthyl-2,4,6 cyclohexadiène-2,5 one.

Le taux de transformation du triméthyl-2,4,6 phénol est de 94,8% et le rendement en hydroxy-4 triméthyl-2,4,6 cyclohexadiène-2,5 one est de 45,6% par rapport au triméthyl-2,4,6 phénol transformé.

*Exemple 5:*

Dans l'appareil décrit dans l'exemple 1, on introduit 2,6 g de bioxyde de manganèse (25 m.moles), 170 cm³ d'eau et 30 g d'acide sulfurique concentré (d = 1,83). On refroidit à 0°C puis on ajoute 100 cm³ d'éther isopropylique. On ajoute ensuite, en 20 minutes, 3,4 g de triméthyl-2,4,6 phénol (25 m.moles) en solution dans 60 cm³ d'éther isopropylique. L'ampoule de coulée est rincée par 25 cm³ d'éther isopropylique. Après 4 heures 30 d'agitation à une température comprise entre 0 et 5°C, le mélange réactionnel est décanté et la couche aqueuse est extraite par 3 fois 20 cm³ d'éther isopropylique. Dans les phases organiques réunies, on dose, par chromatographie en phase gazeuse, 9,1 m.moles de triméthyl-2,4,6 phénol et 9,2 m.moles d'hydroxy-4 triméthyl-2,4,6 cyclohexadiène-2,5 one.

Le taux de transformation du triméthyl-2,4,6 phénol est de 63,6% et le rendement en hydroxy-4 triméthyl-2,4,6 cyclohexadiène-2,5 one est de 58,1% par rapport au triméthyl-2,4,6 phénol transformé.

*Exemple 6:*

Dans l'appareil décrit à l'exemple 1, on introduit 170 cm³ d'eau et 30 g d'acide sulfurique concentré (d = 1,83), 160 cm³ d'éther isopropylique et 3,4 g de triméthyl-2,4,6 phénol (25 m.moles). On refroidit à 0°C puis on ajoute, en 4 heures 30, 2,6 g de bioxyde de manganèse (25 m.moles). Après 2 heures d'agitation après la fin de l'addition, le mélange réactionnel est décanté et la couche aqueuse est extraite par 3 fois 25 cm³ d'éther isopropylique. Dans les phases organiques réunies, on dose, par chromatographie en phase gazeuse, 9,9 m.moles de triméthyl-2,4,6 phénol et 6,8 m.moles d'hydroxy-4 triméthyl-2,4,6 cyclohexadiène-2,5 one.

Le taux de transformation du triméthyl-2,4,6 phénol est de 65,6% et le rendement en hydroxy-4 triméthyl-2,4,6 cyclohexadiène-2,5 one est de 45% par rapport au triméthyl-2,4,6 phénol transformé.

*Exemple 7:*

Dans l'appareil décrit à l'exemple 1, on introduit 2,62 g de bioxyde de manganèse (25 m.moles), 170 cm³ d'eau et 30 g d'acide sulfurique concentré (d = 1,83). On refroidit à 0°C puis on ajoute, en 20 minutes, 3,4 g de triméthyl-2,4,6 phénol en solution dans 10 cm³ d'acide acétique. Après 2 heures 30 d'agitation à une température comprise entre 0 et 5°C, on ajoute 50 cm³ d'éther isopropylique.

On extrait par 4 fois 30 cm³ d'éther isopropylique. Les phases organiques sont réunies puis lavées par 20 cm³ d'eau. Dans les phases organiques, on dose, par chromatographie en phase gazeuse, 8,9 m.moles de triméthyl-2,4,6 phénol et 5,9 m.moles d'hydroxy-4 triméthyl-2,4,6 cyclohexadiène-2,5 one.

Le taux de transformation du triméthyl-2,4,6 phénol est de 64,4% et le rendement en hydroxy-4 triméthyl-2,4,6 cyclohexadiène-2,5 one est de 36,6% par rapport au triméthyl-2,4,6 phénol transformé.

*Exemple 8:*

Dans un appareillage identique à celui décrit dans l'exemple 4, on introduit 184 cm³ d'eau, 3,4 g d'acide sulfurique à 100% (d = 1,83), 3,9 g de bioxyde de manganèse (37,5 m.moles) et 20 cm³ de tétrachlorure de carbone. On refroidit le mélange à 0°C puis on ajoute rapidement 3,45 g de triméthyl-2,4,6 phénol (25 m.moles) dans 50 cm³ de tétrachlorure de carbone. Le mélange réactionnel est agité pendant 4 heures à une température comprise entre 0 et 5°C. Après filtration et décantation, la couche aqueuse est extraite par le tétrachlorure de carbone.

Dans les phases organiques réunies on dose:
— 4,7 m.moles de triméthyl-2,4,6 phénol, et
— 7,7 m.moles d'hydroxy-4 triméthyl-2,4,6 cyclohexadiène-2,5 one.

Le taux de transformation du triméthyl-2,4,6 phénol est de 81% et le rendement en hydroxy-4 triméthyl-2,4,6 cyclohexadiène-2,5 one par rapport au triméthyl-2,4,6 phénol ayant réagi est de 38%.

*Exemple 9:*

Dans un appareillage identique à celui décrit dans l'exemple 4, mais en utilisant un ballon de 1 litre, on introduit 3,96 g de bioxyde de manganèse, 87 g d'acide perchlorique à 70%, 143 g d'eau et 187 g de tétrachlorure de carbone. On refroidit le mélange à 0°C puis on ajoute 3,45 g de triméthyl-2,4,6 phénol en solution dans 50 cm³ de tétrachlorure de carbone. Le mélange réactionnel est agité pendant 4 heures entre 0 et 5°C. Après traitement habituel, le dosage des phases organiques montre que le taux de transformation du triméthyl-2,4,6 phénol est de 81% et que le rendement en hydroxy-4 triméthyl-2,4,6 cyclohexadiène-2,5 one par rapport au triméthyl-2,4,6 phénol ayant réagi est de 20%.

*Exemple 10:*

Dans un ballon de 500 cm³ muni d'une agitation centrale, d'un réfrigérant ascendant et d'un thermomètre, on introduit 3,9 g de bioxyde de manganèse, 30 g d'acide sulfurique à 100% (d = 1,83), 170 cm³ d'eau et 3,4 g de triméthyl-2,4,6 phénol. Le mélange réactionnel est agité pendant 3 heures 50 à une température comprise entre 0 et 5°C puis est extrait par 4 fois 50 cm³ de chlorure de méthylène. Le dosage des phases organiques réunies montre que le taux de transformation du triméthyl-2,4,6 phénol est de 93% et que le rendement en hydroxy-4 triméthyl-2,4,6 cyclohexadiène-2,5 one par rapport au triméthyl-2,4,6 phénol transformé est de 62%.

*Exemple 11:*

Dans un ballon de 500 cm³ muni d'une agitation centrale, d'un réfrigérant ascendant et d'un thermomètre, on introduit 3,9 g de bioxyde de manganèse, 59,4 g d'acide méthanesulfonique, 186 cm³ d'eau et 20 cm³ de chlorure de méthylène. Ce mélange est refroidi à 0°C puis on ajoute rapidement une solution de 3,4 g de triméthyl-2,4,6 phénol dans 30 cm³ de chlorure de méthylène. Le mélange réactionnel est agité pendant 1 heure entre 0 et 5°C.

Après décantation et extraction de la phase aqueuse par 4 fois 50 cm³ de chlorure de méthylène, le dosage des phases organiques réunies montre que le taux de transformation du triméthyl-2,4,6 phénol est de 93% et que le rendement en hydroxy-4 triméthyl-2,4,6 cyclohexadiène-2,5 one par rapport au triméthyl-2,4,6 phénol ayant réagi est de 50%.

*Exemple 12:*

Dans un appareillage identique à celui décrit dans l'exemple 1, on introduit 6,76 g de sulfate de manganèse monohydraté, 170 cm³ d'eau, 30 g d'acide sulfurique à 100% (d = 1,83) et 1,58 g de permanganate de potassium. Au mélange refroidi à 0°C, on ajoute rapidement 3,42 g de triméthyl-2,4,6 phénol en solution dans 50 cm³ de chlorure de méthylène. Le mélange réactionnel est agité pendant 2 heures 40 à 0°C. Après traitement habituel, le dosage des phases organiques réunies montre que le taux de transformation du triméthyl-2,4,6 phénol est de 61% et que le rendement en hydroxy-4 triméthyl-2,4,6 cyclohexadiène-2,5 one par rapport au triméthyl-2,4,6 phénol ayant réagi est de 20%.

*Exemple 13:*

Dans un appareillage identique à celui décrit dans l'exemple 1, on introduit 170 cm³ d'eau, 30 g d'acide sulfurique concentré (d = 1,83), 3,9 g de bioxyde de manganèse (37,1 m.moles) et 20 cm³ de chlorure de méthylène. On ajoute ensuite, en 10 minutes, 3,4 g de triméthyl-2,4,6 phénol en solution dans 20 cm³ de chlorure de méthylène. Le mélange est maintenu à 25°C pendant 1 heure 20 minutes. Le mélange réactionnel est ensuite décanté et la phase aqueuse est extraite par 3 fois 50 cm³ de chlorure de méthylène. Dans les phases organiques réunies, on dose, par chromatographie en phase gazeuse:
— 4,3 m.moles de triméthyl-2,4,6 phénol,
— 14,1 m.moles d'hydroxy-4 triméthyl-2,4,6 cyclohexadiène-2,5 one.

Le taux de transformation du triméthyl-2,4,6 phénol est de 82,8%.

Le rendement en hydroxy-4 triméthyl-2,4,6 cyclohexadiène-2,5 one est de 68,1% par rapport au triméthyl-2,4,6 phénol transformé.

*Exemple 14:*

Dans un appareillage identique à celui décrit dans l'exemple 1, on introduit 89 cm³ d'eau, 19 g d'acide sulfurique concentré (d = 1,83) et 7,9 g de bioxyde de manganèse. Le mélange est refroidi à une température voisine de 0°C. On ajoute ensuite, en 2 minutes environ, 6,8 g de triméthyl-2,4,6 phénol en solution dans 100 cm³ de chlorure de méthylène. On ajoute alors, en 15 minutes, 3,9 g d'acide sulfurique concentré puis, en 75 minutes, 3,9 g d'acide sulfurique concentré. L'agitation est poursuivie pendant encore 30 minutes. Le mélange réactionnel est ensuite décanté et la phase aqueuse est extraite par 4 fois 50 cm³ de chlorure de méthylène. Dans les phases organiques réunies, on dose, par chromatographie en phase vapeur:
— 0,4 m.mole de triméthyl-2,4,6 phénol,
— 36,5 m.moles d'hydroxy-4 triméthyl-2,4,6 cyclohexadiène-2,5 one.

Le taux de transformation du mésitol est de 99,2%.

Le rendement en hydroxy-4 triméthyl-2,4,6 cyclohexadiène-2,5 one est de 73,4% par rapport au triméthyl-2,4,6 phénol transformé.

## Revendications

1. Procédé de préparation de l'hydroxy-4 triméthyl-2,4,6 cyclohexadiène-2,5 one, caractérisé en ce que l'on oxyde le triméthyl-2,4,6 phénol par un dérivé du manganèse de valence supérieure à 2 en milieu acide aqueux éventuellement en présence d'un solvant organique et isole le produit obtenu.

2. Procédé selon la revendication 1, caractérisé en ce que le dérivé du manganèse est choisi parmi le bioxyde de manganèse, le permanganate de potassium ou le sulfate manganique ou leurs mélanges.

3. Procédé selon la revendication 1, caractérisé en ce que le milieu acide aqueux est constitué par de l'acide sulfurique, de l'acide perchlorique ou des acides sulfoniques en solution aqueuse.

4. Procédé selon la revendication 1, caractérisé en ce que le pH de la phase aqueuse est compris entre −0,8 et +0,5.

5. Procédé selon la revendication 1, caractérisé en ce que, lorsque l'on opère en présence d'un solvant organique, celui-ci est choisi parmi les hydrocarbures aromatiques, aliphatiques ou cycloaliphatiques, les éthers, les esters, les solvants chlorés ou les acides organiques aliphatiques.

6. Procédé selon la revendication 1, caractérisé en ce que l'oxydation est effectuée à une température comprise entre −10°C et la température d'ébullition du mélange réactionnel.

7. Procédé selon la revendication 1, caractérisé en ce que, lorsque l'on utilise le bioxyde de manganèse, le rapport molaire entre le bioxyde de manganèse et le triméthyl-2,4,6 phénol est compris entre 1 et 2.

## Claims

1. Process for the preparation of 4-hydroxy-2,4,6-trimethyl-2,5-cyclohexadienone, characterized in that 2,4,6-trimethylphenol is oxidized by a manganese derivative of valency greater than 2 in aqueous acidic medium and optionally in the presence of an organic solvent, and the product obtained is isolated.

2. Process according to Claim 1, characterized in that the manganese derivative is chosen from manganese dioxide, potassium permanganate or manganic sulphate, or mixtures of these.

3. Process according to Claim 1, characterized in that the aqueous acidic medium consists of sulphuric acid, perchloric acid or sulphonic acids in aqueous solution.

4. Process according to Claim 1, characterized in that the pH of the aqueous phase is between −0.8 and +0.5.

5. Process according to Claim 1, characterized in that when the procedure is carried out in the presence of an organic solvent, the latter is chosen from the aromatic, aliphatic or cycloaliphatic hydrocarbons, ethers, esters, chlorinated solvents or aliphatic organic acids.

6. Process according to Claim 1, characterized in that the oxidation is performed at a temperature between −10°C and the boiling point of the reaction mixture.

7. Process according to Claim 1, characterized in that when manganese dioxide is used, the mole ratio between the manganese dioxide and 2,4,6-trimethylphenol is between 1 and 2.

## Patentansprüche

1. Verfahren zur Herstellung von 4-Hydroxy-2,4,6-trimethyl-2,5-cyclohexadienon, dadurch gekennzeichnet, dass man 2,4,6-Trimethylphenol mit einem Derivat von Mangan mit einer Wertigkeit grösser 2 in wässerigem, saurem Milieu gegebenenfalls in Gegenwart eines organischen Lösungsmittels oxidiert und das erhaltene Produkt isoliert.

2. Verfahren nach Anspruch 1, dadurch gekennzeichnet, dass das Manganderivat ausgewählt ist aus Mangandioxid, Kaliumpermanganat oder Mangan(III)sulfat oder deren Mischungen.

3. Verfahren nach Anspruch 1, dadurch gekennzeichnet, dass das wässerige saure Milieu aus Schwefelsäure, Perchlorsäure oder Sulfonsäuren in wässeriger Lösung besteht.

4. Verfahren nach Anspruch 1, dadurch gekennzeichnet, dass der pH-Wert der wässerigen Phase −0,8 bis +0,5 beträgt.

5. Verfahren nach Anspruch 1, dadurch gekennzeichnet, dass das organische Lösungsmittel, falls man in seiner Gegenwart arbeitet, ausgewählt ist aus den aromatischen, aliphatischen oder cycloaliphatischen Kohlenwasserstoffen, den Äthern, den Estern, den chlorierten Lösungsmitteln oder den aliphatischen organischen Säuren.

6. Verfahren nach Anspruch 1, dadurch gekennzeichnet, dass die Oxidation bei einer Temperatur zwischen −10°C und der Siedetemperatur der Reaktionsmischung ausgeführt wird.

7. Verfahren nach Anspruch 1, dadurch gekennzeichnet, dass bei Verwendung von Mangandioxid das molare Verhältnis von Mangandioxid zu 2,4,6-Trimethylphenol zwischen 1 und 2 liegt.